# EUROPEAN PATENT SPECIFICATION

(11) **EP 1 670 923 B1**
(45) Date of publication and mention of the grant of the patent: **10.09.2014**
(21) Application number: 04774724.1
(22) Date of filing: 24.09.2004
(51) Int. Cl.: A61K 38/44

(54) **METHOD FOR MASS PRODUCTION OF HUMAN FOLLICLE STIMULATING HORMONE**
VERFAHREN ZUR MASSENPRODUKTION VON MENSCHLICHEM FOLLIKELSTIMULIERENDEM HORMON
PROCEDE DE PRODUCTION DE MASSE D'HORMONE FOLLICULOSTIMULANTE HUMAINE

(30) Priority: 02.10.2003 KR 2003068641
(43) Date of publication of application: 21.06.2006
(73) Proprietor: Genexine, Inc., Bundang-gu, Seongnam-si Gyeonggi-do 463-400 (KR)
(72) Inventor: YANG, Se Hwan, Gyeongsangbuk-do 790-751 (KR); SUNG, Young Chul, 101-601 Hyundai Edong Hometown, Pohang-si, Gyeongsangbuk-do 790-310 (KR); NA, Kyu-Heum, 312-1303 Cheongmyeong Dongshin Apt., Suwon-si, Kyunggi-do 442-470 (KR); LEE, Sung Hee, 101-306 Nakseongdae Hyundai Apt., Seoul 151-815 (KR); KIM, Won Bae, 1801 Daelim Acrovill Apt., Seoul 135-270 (KR)
(74) Representative: Brady, Paul Andrew
(86) International application number: PCT/KR2004/002474
(87) International publication number: WO 2005/030969

(56) References cited:
- WO-A-02/02783
- WO-A-03/064677
- WO-A1-99/57263
- US-A- 6 110 707
- US-A1- 2001 007 757
- DATABASE WPI Week 199729 Derwent Publications Ltd., London, GB; AN 1997-314236 XP002446173 & JP 09 121885 A (DENKI KAGAKU KOGYO KK) 13 May 1997 (1997-05-13)
- SUGAHARA T ET AL: "EXPRESSION OF BIOLOGICALLY ACTIVE FUSION GENES ENCODING THE COMMON ALPHA SUBUNIT AND EITHER THE CGBETA OR FSHBETA SUBUNITS: ROLE OF A LINKER SEQUENCE" MOLECULAR AND CELLULAR ENDOCRINOLOGY, AMSTERDAM, NL, vol. 125, 1996, pages 71-77, XP002057265 ISSN: 0303-7207
- JAMESON J L ET AL: "Human follicle-stimulating hormone beta-subunit gene encodes multiple messenger ribonucleic acids." MOLECULAR ENDOCRINOLOGY (BALTIMORE, MD.) SEP 1988, vol. 2, no. 9, September 1988 (1988-09), pages 806-815, XP008082171 ISSN: 0888-8809
- GEBERT A. ET AL.: 'Expression of FSH in CHO cells. I. Comparison of promoter types and effects of their respective inducers' CYTOTECHNOLOGY vol. 14, no. 1, 1994, pages 39 - 45, XP008067080
- MANNAERTS B. ET AL.: 'Comparative in vitro and in vivo studies on the biological characteristics of recombinant human follicle-stimulating hormone' ENDOCRINOLOGY vol. 129, no. 5, 1991, pages 2623 - 2630, XP008067081
- HEUM NA KYU ET AL: "PURIFICATION AND CHARACTERIZATION OF RECOMBINANT HUMAN FOLLICLE STIMULATING HORMONE PRODUCED BY CHINESE HAMSTER OVARY CELLS", JOURNAL OF MICROBIOLOGY AND BIOTECHNOLOGY, KOREAN SOCIETY FOR APPLIED MICROBIOLOGY, SEOUL, KR, vol. 15, no. 2, 1 April 2005 (2005-04-01), pages 395-402, XP008079893, ISSN: 1017-7825
- MIZUGUCHI H ET AL: "IRES-dependent second gene expression is significantly lower than cap-dependent first gene expression in a bicistronic vector", MOLECULAR THERAPY, NATURE PUBLISHING GROUP, GB, vol. 1, no. 4, 1 April 2000 (2000-04-01), pages 376-382, XP002213078, ISSN: 1525-0016, DOI: 10.1006/MTHE.2000.0050

## Description

### Technical Field

The present invention relates to a method for mass-production of human follicle stimulating hormone, more precisely, to an expression vector containing human follicle stimulating hormone gene, a transformant transfected with the expression vector and a method for mass-production of human follicle stimulating hormone by using the same.

### Background Art

Follicle stimulating hormone (referred as "FSH" hereinafter), having a molecular weight of 32,600 Da, is a glycoprotein secreted in anterior pituitary, more precisely, a heterodimeric glycoprotein in which alpha and beta subunits are combined each other by a noncovalent bond. Heterodimeric glycoproteins are exemplified by FSH, luteinizing hormone (referred as "LH" hereinafter) which is a pituitary glycoprotein, human chorionic gonadotropin (referred as "hCG" hereinafter) which is a placental glycoprotein, thyroid stimulating hormone (referred as "TSH" hereinafter), factor VIII and IL-12, etc. The alpha subunit of FSH is very similar in structure and in immunological aspects to LH, TSH and hCG. But, the beta subunit is different from every hormone, meaning that each hormone has unique biological and immunological characteristics owing to the different properties of beta subunit (Pierce. J.G and Parsons. T.F., Ann. Rev. Biochem., 50:465-495, 1981). In particular, human FSH (hFSH) stimulates follicle of an ovary to make the follicle be growing, leading to the ovulation. Thus, it has been in clinical use for the treatment of sterility. FSH is obtained from urine of pregnant women, but the obtainable amount is very small and the separation is difficult. Therefore, it is urgently required to develop a method for mass-production of the FSH by using a genetic recombinant technique.

US6,110,707 discloses methods of engineering cells for high level expression of a variety of proteins including peptide hormones , such as human follicle-stimulating hormone, and methods of large scale protein production.

In order to prepare a transformed cell line expressing a glycoprotein hormone by taking advantage of a genetic recombinant technique, *E. coli,* yeast, insect cells or animal cells can be used as a host cell. When the selection of a host cell is made to prepare a glycoprotein, glycosylation capacity or glycosylation system must be considered first because every host cell has a different glycosylation capacity or glycosylation system. Especially to prepare a human FSH, it is required to select a host cell with an excellent glycosylation capacity because glucose plays an important role in *in vivo* activity of hormone. For example, among the above-mentioned candidates for a host cell, *E*. *coli* has no glycosylation capacity at all. Although yeast or insect cells have a little glycosylation capacity, it is too low to produce a complex type glycoprotein such as human FSH, or it can only produce high mannose type glycoprotein. Therefore, they are not promising candidates for a host cell. Even though mammalian cells have a very good glycosylation capacity or glycosylation system, the level or the type of glycosylation varies from the characteristics of each cell line. So, in order to produce a glycoprotein such as hFSH available for the treatment of a disease, such a mammalian cell line that does not cause antigenicity but is safe and has high in *vivo* activity must be selected as a host cell.

It is also essential to develop an efficient expression vector for the mass-production of a heterodimeric glycoprotein such as FSH, LH, hCG, TSH, factor VIII and IL-12 by using animal cells. These are the examples of a constituent of such expression vector; promoter playing an important role in controlling the gene expression, intron enabling the increase of transcription efficiency of a gene by stabilizing mRNA, polyadenylation motif (pA) enabling the increase of the stability of mRNA, and selection marker, etc.

Thus, the present inventors prepared an expression vector highly and stably expressing human FSH, and then completed this invention by confirming that FSH could be mass-produced stably by transfecting a host cell having an excellent glycosylation capacity with the prepared expression vector.

### Disclosure

### Technical Problem

It is an object of the present invention to provide an expression vector containing human follicle stimulating hormone gene, a transformant transfected with the expression vector and a method for mass-production of human follicle stimulating hormone by using the same.

### Technical Solution

In order to achieve the above object, the present invention provides a recombinant expression vector containing human FSH gene.

The present invention also provides a transformant transfected with the expression vector.

The present invention further provides a method for mass-production of human follicle stimulating hormone by using the transformant.

Hereinafter, the present invention is described in detail.

The present invention provides a recombinant expression vector containing human FSH gene.

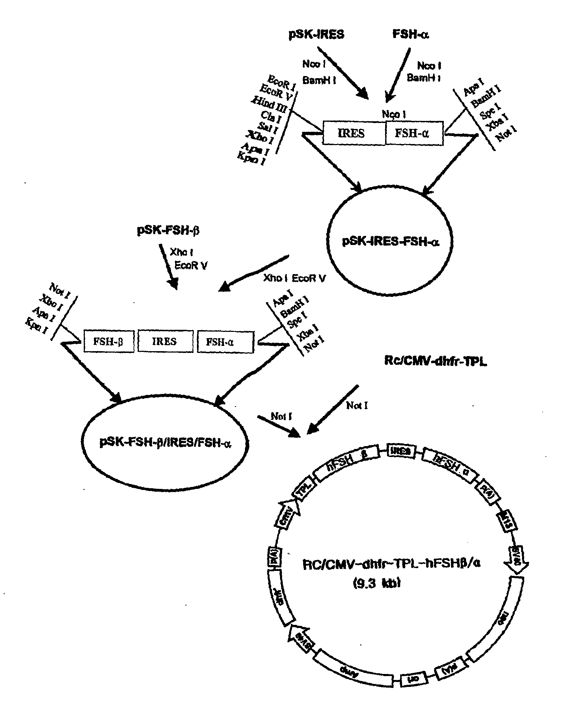

In order to mass-produce heterodimeric glycoprotein such as FSH, LH, hCG, TSH, factor VIII and IL-12 using animal cells, it is essential to develop an efficient expression vector. Thus, the present invention provides a highly efficient expression vector for mass-production of human FSH, considering all the factors involved.
1) A gene coding hFSH is composed of two subunits (alpha and beta subunits) which are transcribed from different genes. In the present invention, IRES (internal ribosomal entry site) of EMCV (encephalomyocarditis virus) was used in order to prepare a heterodimeric glycoprotein by expressing two different glycoproteins in the same cell. Then the resultant was inserted in between alpha subunit gene and beta subunit gene of hFSH to make those genes be expressed simultaneously. So, it is preferred for a gene coding hFSH included in the expression vector of the present invention to contain FSH alpha subunit gene represented by SEQ. ID. No 1 and FSH beta subunit gene represented by SEQ. ID. No 2, and it is more preferred for the gene coding hFSH to have IRES sequence, represented by SEQ. ID. No 7, inserted in between FSH alpha subunit gene and FSH beta subunit gene in order to induce simultaneous expression of both subunit genes.
2) A promoter plays an important role in the regulation of gene expression. So, an expression vector of the present invention was prepared to contain a promoter gene. A promoter included in the expression vector of the present invention is preferably the promoter of early gene of cytomegalovirus (CMV) represented by SEQ. ID. No 8 which has been known as the most powerful promoter.
3) Mammalian genes contain intron. The expression level is remarkably increased in the presence of intron (Korb et al., Nucleic Acids Res, 25:5901-5908, 1993). This might be because that intron can stabilize mRNA and increase transcription efficiency. Thus, an expression vector of the present invention was designed to have intron sequence, and the intron sequence, at that time, was preferably tripartite leader sequence (referred as "TPL" hereinafter) of adenovirus represented by SEQ. ID. No 9.
4) In order to increase the stability of mRNA, polyadenylation motif (pA) is important (Drummond et al., Nucleic Acids Res, 25:7375-7394, 1985). The expression vector of the present invention includes such polyadenylation motif, and the polyadenylation motif is preferred to be polyadenylation motif of early gene of SV40 virus, represented by SEQ. ID. No 13, and/or polyadenylation motif of bovine growth hormone (BGH) gene, represented by SEQ. ID. No 14. In the preferred embodiment of the present invention, an expression vector was prepared by locating BGH pA behind FSH alpha gene, SV40 pA behind Neo gene, and SV40 pA gene behind dihydrofolate reductase (referred as "DHFR") gene (see FIG. 1).
5) A selection marker gene is necessary to select cell lines that can over-express FSH after being transfected with the expression vector of the present invention. Thus, the expression vector of the present invention was designed to include a selection marker gene, and for the selection marker, DHRF gene, a selection marker represented by SEQ. ID. No 12, was preferably used to facilitate selection of a cell line from CHO/dhrf⁻ cells which were Chinese hamster ovary (CHO) originated cell line. CHO/dhfr⁻ cell line has damaged DHFR gene, indicating that its' nucleic acid synthesizing process is incomplete. But, when the cell is transfected with FSH expression vector of the present invention containing DHFR gene, nucleic acid synthesis can be completed. Thus, it is possible to select those cells expressing FSH by the introduction of FSH expression vector.

Taking all the above-mentioned factors into consideration, the present inventors prepared a recombinant expression vector to express human FSH that includes 1) a gene coding human FSH, 2) a promoter sequence, 3) an intron sequence, 4) a polyadenylation motif sequence, and 5) a selection marker gene.

Thus the present invention provides an expression vector to express human FSH comprising a CMV promoter sequence-TPL sequence-a gene encoding human FSH beta subunit-IRES sequence-a gene encoding human FSH alpha subunit-a polyadenylation motif sequence, sequentially; and a DHFR gene.

In the present invention, in order to express both FSH alpha and beta subunits simultaneously in the same cell, IRES was inserted in between FSH alpha subunit gene and FSH beta subunit gene. And the expression efficiency of hFSH was maximized by using a promoter of early gene of cytomegalovirus that has been known as the most powerful promoter. Also, adenovirus tripartite leader sequence (TPL) containing intron to increase the stability and transcription efficiency and polyadenylation motif of early gene of SV40 virus as well as polyadenylation motif of bovine growth hormone gene were all included in the vector of the present invention. In addition, DHFR gene was included as a selection marker to facilitate the selection of cell lines over-expressing FSH from the cells transfected with the expression vector of the present invention.

The present inventors prepared an expression vector containing all the above-mentioned elements and named it 'Rc/CMV-dhfr-TPL-hFSH beta/alpha'.

The expression vector of the present invention was prepared for the high-expression of human FSH gene, a heterodimeric glycoprotein. And the vector of the invention is very efficient one enabling mass-production of a heterodimeric glycoprotein. It is well known to the people in this field that instead of human FSH gene, any other heterodimeric glycoprotein such as LH, hCG, TSH, factor VIII and IL-12 gene can be included in the expression vector and the resultant vector can be effectively used for the mass-production of a protein coded from the above genes.

The present invention also provides a transformant transfected with the expression vector.

In order to prepare a cell line high-expressing human FSH, which is highly activated in a human body, the present inventors selected Chinese hamster ovary (referred as "CHO" hereinafter) cells among many mammalian cell lines as a host cell line because they have a satisfactory glycosylation capacity or glycosylation system, do not induce antigenicity *in vivo,* are safe and have a carbohydrate structure showing a high *in vivo* activity.

Nucleic acid biosynthesizing processes occurring in CHO/dhfr⁻ cells, originated from CHO, are incomplete because DHFR gene therein is damaged. But, nucleic acid biosynthesis is fully recovered in CHO cells when they are transfected with the FSH expression vector of the present invention (Rc/CMV-dhfr-TPL-hFSH beta/alpha) that contains DHFR gene. Therefore, it is possible to select those cells harboring the expression vector by culturing CHO cells in a cell culture medium devoid of nucleic acid. And the cells are growing in a culture medium supplemented with methotrexate (MTX), a substrate analogue of DHFR, by the amplification of DHFR gene in cells. At this time, the amplification of FSH gene is also performed because FSH expression vector of the present invention contains DHFR gene. Thus, adding MTX, a substrate analogue of DHFR, into a cell culture medium can improve the expression level of FSH protein in FSH expressing cells.

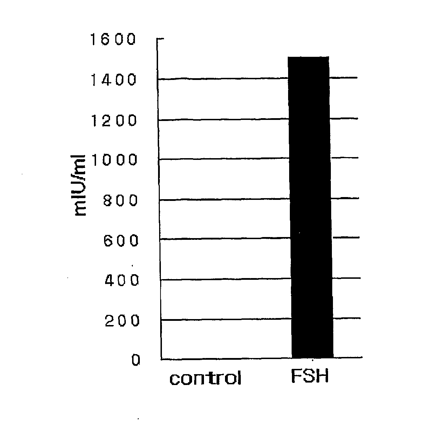

In the present invention, CHO/dhfr⁻ cells were transfected with FSH expressing vector, and clones which were highly expressed in high-concentration of MTX and showed homologous were selected and further cultured, resulting in a cell line in which FSH was stably and highly expressed (see FIG. 4 to FIG. 6). The selected cell line high-expressing FSH was named "DPFC325" which was deposited at Korean Cell Line Research Foundation (KCLRF) on June 20, 2003 (Accession No: KCLRF-BP-00082).

The present invention also provides a method for mass-production of human follicle stimulating hormone by using the transformant.

The method for mass-production of human FSH of the present invention includes the following steps:
1) transfecting host cells with the expression vector described above;
2) selecting recombinant transformants transfected in the step 1);
3) selecting recombinant transformant stably producing FSH from the recombinant transformants selected in the step 2); and
4) obtaining human FSH from the culture of the recombinant transformant selected in the step 3).

In the above method the expression vector preferably includes human FSH coding gene, a promoter sequence, an intron sequence, a polyadenylation motif sequence and a selection marker gene, and is more preferably "Rc/CMV-dhfr-TPL-hFSH beta/alpha" constructed in the present invention.

In step 1), the host cell is preferably CHO/dhfr⁻cell, a CHO originated cell line having incomplete nucleic acid biosynthesizing processes because of the damaged DHFR gene.

In step 3), the recombinant transformant stably producing FSH is preferably "DPFC325" which was deposited at Korean Cell Line Research Foundation (KCLRF) on June 20, 2003 (Accession No: KCLRF-BP-00082).

In step 4), the obtainment of produced FSH is performed by conventional protein purification methods.

### Description of Drawings

FIG. 1 is a schematic diagram showing the preparation process of Rc/CMV-DHFR-TPL-hFSH beta/alpha vector of the present invention,
FIG. 2 shows cDNA nucleotide sequence and amino acid sequence of hFSH-alpha subunit expressed by pSK-FSH-alpha vector of the present invention,
FIG. 3 shows cDNA nucleotide sequence and amino acid sequence of hFSH-beta subunit expressed by pSK-FSH-beta vector of the present invention,
FIG. 4 is a graph showing the expression of human FSH after transfecting COS-7 cells with Rc/CMV-dhfr-TPL-hFSH beta/alpha vector, confirmed by enzyme-linked immunosorbent assay (ELISA),
FIG. 5 is a graph showing the result of enzyme-linked immunosorbent assay (ELISA), confirming the expression level of human FSH in clones that were selected from cells adapted to 1 uM of MTX by limiting dilution,
FIG. 6 is a photograph showing the result of FISH, confirming whether or not human FSH gene is inserted in chromosome of a CHO cell.

### Mode for Invention

Practical and presently preferred embodiments of the present invention are illustrative as shown in the following Examples.

However, it will be appreciated that those skilled in the art, on consideration of this disclosure, may make modifications and improvements within the spirit and scope of the present invention.

### <Example 1> Construction of a recombinant hFSH expression vector (Rc/CMV-dhfr-TPL-hFSH beta/alpha) <1-1> Preparation of human FSH alpha subunit and beta subunit

In order to prepare a structural gene that is essential for the production of a cell line expressing a recombinant human FSH, human FSH alpha subunit and beta subunit genes obtained from human pituitary gland cDNA library were amplified by PCR. More precisely, human pituitary gland cDNA library (cal#HL1139a, Clontech, USA) was used as a substrate, and primers were prepared for PCR with nucleotide sequence of FSH alpha subunit gene represented by SEQ. ID. No 1 and nucleotide sequence of FSH beta subunit gene represented by SEQ. ID. No 2. The primer sequences for PCR amplification of FSH alpha and beta subunit genes were as follows.

### 1) FSH alpha subunit

Sense primer (SEQ. ID. No 3): AGCGCCATGGATTACTACAGAAAATAT (underlined region : Nco I recognition site),

Antisense primer (SEQ. ID. No 4): GGGGGATCCGGGCCCTTAAGATTTGTGATAATTAACA (underlined region : BamH I and Apa I recognition site)

### 2) FSH beta subunit

Sense primer (SEQ. ID. No 5): GGGGGCGGCCGCAGGATGAAGACACTCCAGTTT (underlined region : Not I recognition site),

Antisense primer (SEQ. ID. No 6): GGGGATATCTTATTCTTTCATTTCACC (underlined region : EcoR V recognition site)

The whole fraction of pituitary gland cDNA library was used as a substrate, and each pair of primers (alpha subunit: SEQ. ID. No 3 and No 4, beta subunit: SEQ. ID. No 5 and No 6) were used for PCR. PCR was performed as follows; predenaturation at 94°C for 30 seconds, denaturation at 94°C for 30 seconds, annealing at 57°C for 30 seconds, polymerization at 72°C for 30 seconds, 30 cycles from denaturation to polymerization, and final extension at 72°C for 5 minutes. The same PCR condition as described above was applied to the amplification of both FSH alpha subunit and FSH beta subunit.

### <1-2> Construction of a vector for simultaneous expression of FSH alpha and beta subunit genes

FSH is composed of two subunits, which are FSH alpha subunit and FSH beta subunit. So, in order to construct an expression vector enabling simultaneous expression of both FSH alpha subunit and FSH beta subunit, IRES (internal ribosomal entry site) of EMCV (encephalomyocarditis virus), represented by SEQ. ID. No 7 was introduced, in which beta subunit was located at the upstream and alpha subunit was located at the downstream.

First, FSH beta subunit gene (390 nucleotides) which was amplified in the above example <1-1> was treated with T4 polynucleotide kinase, then cloned into pSK vector (Stratagene, USA), pretreated with Hinc II, by blunt ligation. The resultant vector was named "pSK-FSH-beta". Next, FSH alpha subunit gene (351 nucleotides), which had been amplified in the above example <1-1>, was digested with Nco I and BamH I, then inserted in the location of Nco I and BamH I of pSK-IRES vector (Ha et al., Nat. Biotechnol., 20(4):381-386, 2002) containing EMCV IRES, resulting in "pSK-IRES-FSH-alhpa". FSH-beta DNA fragment, prepared by digesting pSK-FSH beta vector with Xho I and EcoR V, was inserted in pSK-IRES-FSH-alpha vector, which had been digested with Xho I and EcoR V, resulting in the construction of a target vector containing both FSH-alpha subunit gene and FSH-beta subunit gene, which was named "pSK-FSH-alpha/IRES/FSH-beta".

Lastly, a vector was constructed to express FSH-alpha subunit gene and FSH-beta subunit gene simultaneously in animal cells. For the construction, Rc/CMV plasmid (Invitrogen, USA) harboring a very powerful cytomegalovirus (CMV) promoter, represented by SEQ. ID. No 8, was used as a basic vector. In order to increase the level of expression, adenovirus tripartite leader sequence (TPL), represented by SEQ. ID. No 9, was inserted in pTV2 vector (Lee et al., J. Virol., 72(10):8430-8436, 1998). More precisely, pTV2 vector was used as a template and TPL gene was amplified by PCR using the below primers under the same PCR condition as described in the example <1-1>.

The primer sequences for the amplification of TPL gene are as follows.

Sense primer (SEQ. ID. No 10) : GATATCGATACTCTCTTCC,

Antisense primer (SEQ. ID. No 11): GCGTCGACCTGCAGTTGGACCTGGGAG (underlined region : Sal I recognition site)

The amplified TPL gene was treated with T4 polynucleotide kinase, which was then inserted in pSK vector (Stratagene, USA) predigested with EcoR V. The resultant product was digested with Sal I, so that the location of Sal I in pSK vector which was corresponding to the area of 5' of TPL gene and the location of Sal I in 3' of TPL gene were restricted, resulting in the separation of TPL gene. The separated gene was treated with Klenow enzyme to make it have blunt ends. After digesting with Hind III, Rc/CMV vector (Invitrogen, USA) was also treated with Klenow enzyme to make it have blunt ends, too. The treated vector was linked to the separated TPL gene by the action of ligase, resulting in RcCMV-TPL vector. For the amplification of the gene expression, pSV2-dhfr vector (ATCC 37146) containing mouse dihydrofolate reductase (DHFR) gene, represented by SEQ. ID. No 12, and SV40 pA gene, represented by SEQ. ID. No 13, in the rear of the above gene was digested with Pvu II, then BamH I linker (SEQ. ID. No 15) was inserted in the position. The resultant product was digested with BamH I, resulting in 1.8 kb sized gene containing DHFR gene and SV40 pA gene. The gene was inserted in RcCMV-TPL vector which had been digested with Bgl II, and the final vector was named "Rc/CMV-dhfr-TPL". PSK-FSH-alpha/IRES/FSH-beta vector was digested with Not I to prepare FSH-alpha/IRES/FSH-beta fragment. The fragment was inserted in the location of Not I of Rc/CMV-dhfr-TPL vector to prepare an expression vector for high-expression of FSH. The product was named "Rc/CMV-dhfr-TPL-hFSH beta/alpha" (FIG. 1). DNA sequencing of FSH-alpha subunit gene and FSH-beta subunit gene in Rc/CMV-dhfr-TPL-hFSH beta/alpha plasmid was performed by the method of Sanger *et al*. (Sanger et al., Proc. Natl. Acad. Sci. USA, 74:5463-5467, 1977).

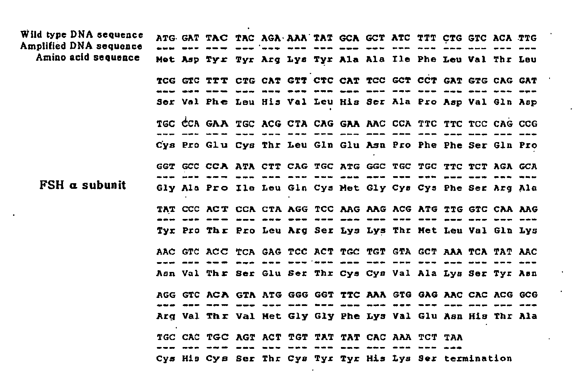

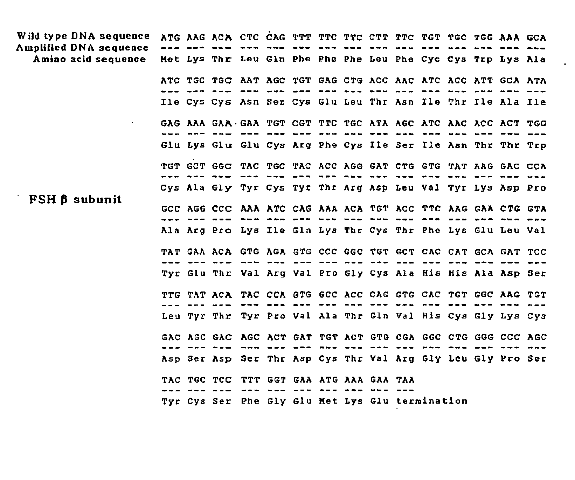

As a result, DNA sequences of FSH-alpha subunit and FSH-beta subunit that were inserted in the expression vector of the present invention were confirmed to be identical with the reported DNA sequences of FSH-alpha subunit and FSH-beta subunit, indicating that the expression vector of the present invention was correctly constructed (FIG. 2 and FIG. 3).

### <Example 2> Preparation of a cell line expressing human FSH

A cell line was transfected with the recombinant human FSH expression vector (Rc/CMV-dhfr-TPh-hFSH beta/alpha), constructed in the above example 1, to investigate the expression of recombinant human FSH. Particularly, COS-7 cells (ATCC, catalog # CRL-1651), being under culture, were inoculated into 60 mm dish plates (5 x 10⁵ cells/plate), followed by further culture for 18 hours. The culture medium (DMEM + 10% FBS) was replaced with a fresh medium (4.5 ml). Then, about 4 hours later, the cells were transfected with a FSH expression vector Rc/CMV-dhfr-TPL-hFSH beta/alpha plasmid or a control vector Rc/CMV-dhfr-TPL plasmid by means of CaPO₄ coprecipitation. More precisely, 10 *µ*g of each DNA was mixed with pure distilled water, making the final volume 225 *µ*ℓ. Then, 25 *µ*ℓ of 2.5 M CaCl₂ solution was added and mixed by vortexer. 250 *µ*ℓ of 2x HBS solution (280 mM NaCl, 10 mM KCl, 1.5 mM Na₂HPO₄2H₂O, 12 mM dextrose, 50 nM HEPES) was slowly dropped drop by drop into a tube containing the DNA. Reaction was induced for 10 minutes at room temperature. The mixture was transferred onto a cell culture dish, followed by further culture for 6 hours. The dish was washed with PBS (phosphate buffered saline) three times and then a fresh medium was supplemented, followed by further culture for 48 more hours.

### <Example 3> Confirmation of hFSH expression by ELISA

Temporarily over-expressed FSH was quantified by using the culture medium of COS-7 cells transfected with Rc/CMV-dhfr-TPL-hFSH beta/alpha plasmid or Rc/CMV-dhfr-TPL (control plasmid) in the above example 2. ELISA (enzyme-linked immunosorbent assay) kit (MEDI-CORP, cat# KTSF 3651, Canada) was used to detect FSH, by following the manufacturer's protocol. Particularly, 100 µℓ of the transfected cell culture medium cultured for 48 hours and 100 µℓ of standard solution equipped in the kit were added to the well coated with antihuman FSH, leading to the reaction for 30 minutes on a plate shaker. The wells were washed with washing solution provided by the manufacturer, and then, antihuman FSH antibody linked to HRP (horse radish peroxidase) was added to each well by 100 µℓ, followed by reaction on the plate shaker at room temperature. After 30 minutes of reaction, the plate was washed with washing solution three times. Then, 100 µℓ of substrate solution was added to each well and reaction was induced at room temperature on the plate shaker. 15 minutes later, 100 µℓ of stop solution was added to each well and OD₄₅₀ was measured. Using the standard solution provided by the manufacturer of the kit, standard curve and function were obtained. The amount of FSH was quantified by using the standard curve and the function.

As a result, human FSH was hardly shown in cells transfected with Rc/CMV-dhfr-TPL, a control plasmid, but human FSH was over-expressed in cells transfected with Rc/CMV-dhfr-TPL-hFSH beta/alpha plasmid (FIG. 4).

### <Example 4> Preparation of a CHO cell line expressing hFSH

Rc/CMV-dhfr-TPL-hFSH beta/alpha vector, which was proved in the above example 3 to over-express hFSH, was introduced into Chinese hamster ovary cells (CHO/dhfr⁻) (Urlaub et al., Somat. Cell Mol. Genet., 12:555-566, 1986) having incomplete nucleic acid biosynthesizing process resulted from the damaged DHFR gene, leading to the preparation of a cell line suitable for mass-production of hFSH protein. More precisely, before a hFSH over-expressing vector was introduced, CHO/dhfr⁻ cells were thawed and put in a T-75 culture flask, followed by culture in MEM-alpha medium (GIBCO BRL,

USA) supplemented with HT supplement (GIBCO BRL, USA) and 10% dialyzed fetal bovine serum (FBS) until cell density reached 80-90%. Trypsin was treated to those cells growing with being adhered to the plate. Then, cells were recovered and 5 x 10⁵ cells were plated on 60 mm dish. The cells were transfected with Rc/CMV-dhtr-TPL-hFSH beta/alpha, a FSH expression vector, by means of CaPO₄ coprecipitation explained in the above example 3, followed by further culture for two more days. After the completion of the culture, the transfected CHO/dhfr⁻ cells were diluted consecutively (1/2, 1/5, 1/10, 1/20, 1/50, 1/100, 1/200, 1/500), and then, cultured in HT supplement-free medium on 100 mm dish, during which the primary screening was performed. During screening, the medium was replaced once a week, passage was omitted, and plates with colony formed were selected after 2 to 3 weeks. Colonies were separated from the plates and transferred into a 24-well plate for subculture. About one or two weeks later, cell clones in the 24-well plate were treated with trypsin. Then, cells were recovered and plated again in a 24-well plate (2 x 10⁴ cells/well). 48 hours later, supernatant was obtained to measure the expression level of FSH by using FSH detecting ELISA kit (MEDI-CORP, cat# KTSF 3651, Canada). Cell clones showing higher level of FSH expression were diluted consecutively, which were put in a 100 mm dish for further culture. 20 nM of methotrexate (MTX) was added to the medium for the second screening. Finally, clones high-expressing human FSH were selected with the same identifying processes as used for the primary screening.

### <Example 5> Selection of a CHO cell line expressing hFSH high

CHO/dhfr⁻ cells deficient in DHFR cannot survive in MEM-alpha medium, a minimum essential medium devoid of nucleic acid, because the cells have an incomplete nucleic acid biosynthesizing process. However, recombinant CHO cells in which FSH gene and DHFR gene are inserted, recover nucleic acid biosynthesizing capability, so that the MEM-alpha medium can be used for the selection culture. Thus, in the present invention, in order to select cell lines expressing hFSH high, clones prepared in the above example 4 were cultured in culture media having different MTX concentrations (20 nM to 1 µM). More precisely, the primary selected clones were subcultured in MEM-alpha medium supplemented with 10% of dialyzed FBS, during which the concentration of MTX was increased gradually. Clones were cultured at different MTX concentrations to select specific ones having resistance against MTX. The supernatants of each clone in different culture stages were obtained to measure the expression of FSH, resulting in the second selection of cells expressing FSH high. The expression of FSH was quantified by means of ELISA described in the above example 3.

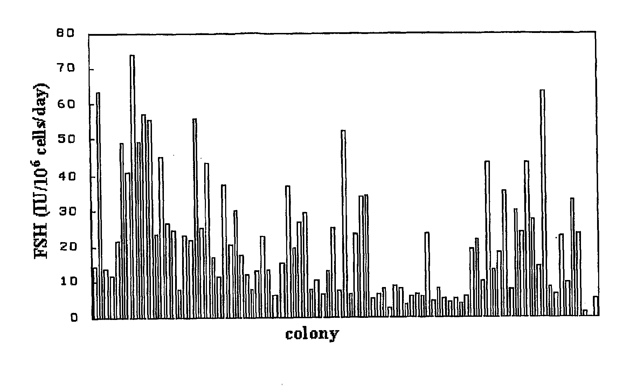

For the third selection, monoclonal selection was performed by means of limiting dilution method to reduce the chance of heterogeneity during MTX amplification. That is, single cells having even FSH expression rate and excellent productivity were separated from all the clones showing irregular FSH expression rates by means of limiting dilution method. Cells were distributed in a 96-well plate by one-two clones per each well, and then the wells inoculated with 1 cell only were selected, followed by further culture for 2 to 3 weeks. From the plate showing colonies formed, cells were separated and sub-cultured to measure the amount of expressed FSH by ELISA. At last, single cells highly expressing FSH were separated (FIG. 5). Among those separated cells through the third selection, a recombinant CHO cell line showing a stable proliferation in the presence of 1 µM MTX and high FSH productivity was selected and named "DPFC325", which was deposited at Korean Cell Line Research Foundation (KCLRF), Seoul, Korea on June 20, 2003 (Accession No : KCLRF-BP-00082 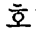 ).

### <Example 6> Confirmation of the insertion of hFSH gene in chromosome by FISH

For the clones used for the production of a protein as a treating agent, genetic stability is so important. In order for a plasmid vector inserted in a cell line to be transmitted to the next generation stably, integration in chromosome of CHO cell is essential. In the present invention, FISH (fluorescent *in situ* hybridization) was performed to investigate whether FSH gene was inserted in chromosome of a recombinant cell line or not. Precisely, metaphase chromosome of CHO cell and nucleus of interphase were hybridized with biotin-labeled probe. The hybridized metaphase chromosome was reacted with avidin conjugated with a fluorescent material, followed by observation.

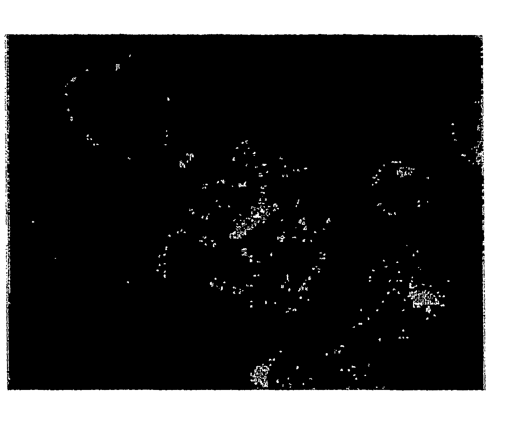

As a result, almost all nuclei of interphase showed reaction color, indicating that FSH gene was successfully inserted in chromosome (FIG. 6).

### Industrial Applicability

As explained hereinbefore, a transformant of the present invention can be effectively used for the treatment of infertility since the transformant can produce human follicle stimulating hormone stably and largely.

### Sequence List Text

Nucleotide sequences represented by SEQ. ID. No 1 to SEQ. ID. No 15 are the ones used for the construction of recombinant hFSH expression vector (Rc/CMV-dhfr-TPL-hFSH beta/alpha) of the example 1.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended claims.

## Claims

1. An expression vector to express human FSH comprising
a CMV promoter sequence-TPL tripartite leader sequence-a gene encoding human FSH beta subunit-IRES sequence-a gene encoding human FSH alpha subunit-a polyadenylation motif sequence, sequentially; and a DHFR gene.

2. The expression vector as set forth in claim 1, wherein the gene encoding human FSH alpha subunit consists of sequences represented by SEQ. ID. No 1, the IRES sequence consists of sequences represented by SEQ. ID. No 7 and the gene encoding human FSH beta subunit consists of sequences represented by SEQ. ID. No 2.

3. The expression vector as set forth in claim 1, wherein the CMV promoter is the promoter of early gene of CMV represented by SEQ. ID. No 8.

4. The expression vector as set forth in claim 1, wherein TPL sequence consists of sequences represented by SEQ. ID. No 9.

5. The expression vector as set forth in claim 1, wherein the polyadenylation motif is a polyadenylation motif of early gene of SV40 virus, represented by SEQ. ID. No 13, and/or a polyadenylation motif of BGH gene, represented by SEQ. ID. No 14.

6. The expression vector as set forth in claim 1, wherein the DHFR gene is a base sequence represented by SEQ. ID. No 12.

7. The expression vector as set forth in claim 1, wherein the vector is RC/CMV-dhfr-TPL-hFSH beta/alpha represented in FIG. 1.

8. A recombinant transformant mass-producing human FSH prepared by introducing the expression vector of claim 1 into host cells.

9. The recombinant transformant as set forth in claim 8, wherein the host cell is a CHO originated cell line harboring damaged DHFR gene.

10. A recombinant transformant DPFC325 deposited under Accession No: KCLRF-BP-00082 mass-producing human FSH prepared by introducing the expression vector of claim 7 into a CHO originated cell line.

11. A method for mass-production of human FSH comprising the following steps:
1) transfecting host cells with the expression vector of claim 1;
2) selecting recombinant transformants transfected in the step 1);
3) selecting recombinant transformant stably producing human FSH from the recombinant transformants selected in the step 2); and
4) obtaining human FSH from the culture of the recombinant transformant selected in the step 3).

12. The method for mass-production of human FSH as set forth in claim 11, wherein the host cell of step 1) is a CHO originated cell line harboring damaged DHFR gene.

## Patentansprüche

1. Expressionsvektor zum Exprimieren von Human-FSH,
der Folgendes aufweist:
nacheinander eine CMV-Promotorsequenz - eine TPL-Sequenz (dreiteilige Leader-Sequenz) - ein Gen, das die β-Untereinheit des Human-FSH codiert - eine IRES-Sequenz - ein Gen, das die α-Untereinheit des Human-FSH codiert - eine Polyadenylierungmotiv-Sequenz; und ein DHFR-Gen.

2. Expressionsvektor nach Anspruch 1,
wobei das Gen, das die α-Untereinheit des Human-FSH codiert, aus Sequenzen besteht, die mit der Sequenzidentifizierungsnr. 1 angegeben werden, die IRES-Sequenz aus Sequenzen besteht, die mit der Sequenzidentifizierungsnr. 7 angegeben werden, und das Gen, das die β-Untereinheit des Human-FSH codiert, aus Sequenzen besteht, die mit der Sequenzidentifizierungsnr. 2 angegeben werden.

3. Expressionsvektor nach Anspruch 1,
wobei der CMV-Promotor der Promotor des frühen Gens von CMV ist, das mit der Sequenzidentifizierungsnr. 8 angegeben wird.

4. Expressionsvektor nach Anspruch 1,
wobei die TPL-Sequenz aus Sequenzen besteht, die mit der Sequenzidentifizierungsnr. 9 angegeben werden.

5. Expressionsvektor nach Anspruch 1,
wobei das Polyadenylierungsmotiv ein Polyadenylierungsmotiv des frühen Gens des SV40-Virus, das mit der Sequenzidentifizierungsnr. 13 angegeben wird, und/oder ein Polyadenylierungsmotiv des BGH-Gens ist, das mit der Sequenzidentifizierungsnr. 14 angegeben wird.

6. Expressionsvektor nach Anspruch 1,
wobei das DHFR-Gen eine Basensequenz ist, die mit der Sequenzidentifizierungsnr. 12 angegeben wird.

7. Expressionsvektor nach Anspruch 1,
wobei der Vektor RC/CMV-dhfr-TPL-hFSH β/α ist, wie er in Fig. 1 angegeben ist.

8. Rekombinante, Human-FSH massenproduzierende Transformante,
die durch Einführen des Expressionsvektors nach Anspruch 1 in Wirtszellen hergestellt ist.

9. Rekombinante Transformante nach Anspruch 8,
wobei die Wirtszelle eine von CHO stammende Zelllinie ist, die ein beschädigtes DHFR-Gen enthält.

10. Rekombinante, Human-FSH massenproduzierende Transformante DPFC325, die unter der Zugangsnr. KCLRF-BP-00082 hinterlegt ist, die durch Einführen des Expressionsvektors nach Anspruch 7 in eine von CHO stammende Zelllinie hergestellt ist.

11. Verfahren für die Massenproduktion von Human-FSH,
das die folgenden Schritte aufweist:
1) Transfizieren von Wirtszellen mit dem Expressionsvektor nach Anspruch 1;
2) Auswählen von rekombinanten Transformanten, die im Schritt 1) transfiziert wurden;
3) Auswählen einer rekombinanten Transformante, die beständig Human-FSH produziert, aus den rekombinanten Transformanten, die im Schritt 2) ausgewählt wurden; und
4) Gewinnen von Human-FSH aus der Kultur der rekombinanten Transformante, die im Schritt 3) ausgewählt wurde.

12. Verfahren zur Massenproduktion von Human-FSH nach Anspruch 11,
wobei die Wirtszelle vom Schritt 1) eine von CHO-stammende Zelllinie ist, die ein beschädigtes DHFR-Gen enthält.

## Revendications

1. Vecteur d'expression pour l'expression de la FSH humaine, comprenant :
une séquence de promoteur de CMV - une séquence leader tripartite TPL - un gène codant pour la sous-unité bêta de la FSH humaine - une séquence IRES - un gène codant pour la sous-unité alpha de FSH humaine - une séquence de motif de polyadénylation, successivement ; et un gène DHFR.

2. Vecteur d'expression suivant la revendication 1, dans lequel le gène codant pour la sous-unité alpha de FSH humaine consiste en séquences représentées par la SEQ ID N° 1, la séquence IRES consiste en séquences représentées par la SEQ ID N° 7 et le gène codant pour la sous-unité bêta de FSH humaine consiste en séquences représentées par la SEQ ID N° 2.

3. Vecteur d'expression suivant la revendication 1, dans lequel le promoteur du CMV est le promoteur de gène précoce du CMV représenté par la SEQ ID N° 8.

4. Vecteur d'expression suivant la revendication 1, dans lequel la séquence TPL consiste en séquences représentées par la SEQ ID N° 9.

5. Vecteur d'expression suivant la revendication 1, dans lequel le motif de polyadénylation est un motif de polyadénylation de gène précoce du virus SV40, représenté par la SEQ ID N° 13, et/ou un motif de polyadénylation du gène BGH, représenté par la SEQ ID N° 14.

6. Vecteur d'expression suivant la revendication 1, dans lequel le gène DHFR est une séquence de bases représentée par la SEQ ID N° 12.

7. Vecteur d'expression suivant la revendication 1, le vecteur étant RC/CMV-dhfr-TPL-hFSH bêta/alpha représenté sur la figure 1.

8. Transformant recombinant produisant en masse de la FSH humaine, préparé en introduisant le vecteur d'expression de la revendication 1 dans des cellules hôtes.

9. Transformant recombinant suivant la revendication 8, la cellule hôte étant une lignée cellulaire issue de cellules CHO portant un gène DHFR lésé.

10. Transformant recombinant DPFC325 déposé sous le numéro de dépôt KCLRF-BP-00082 produisant en masse de la FSH humaine, préparé en introduisant le vecteur d'expression de la revendication 7 dans une lignée cellulaire issue de cellules CHO.

11. Procédé pour la production en masse de FSH humaine comprenant les étapes suivantes :
1) transfection de cellules hôtes avec les vecteurs d'expression de la revendication 1 ;
2) sélection de transformants recombinants transfectés dans l'étape 1 ;
3) sélection d'un transformant recombinant produisant de manière stable de la FSH humaine à partir des transformants recombinants sélectionnés dans l'étape 2 ; et
4) obtention de FSH humaine à partir de la culture du transformant recombinant sélectionné dans l'étape 3.

12. Procédé pour la production en masse de FSH humaine suivant la revendication 11, dans lequel la cellule hôte de l'étape 1 est une lignée cellulaire issue de cellules CHO portant un gène DHFR lésé.


### SequenceListing

<110> ProGen Co Ltd.
<120> Method for mass production of human Follicle Stimulating Hormone
<150> KR 10-2003-0068641
   <151> 2003-10-02
<160> 15
<170> KopatentIn 1.71
<210> 1
   <211> 351
   <212> DNA
   <213> Homo sapiens
<400> 1
   atggattact acagaaaata tgcagctatc tttctggtca cattgtcggt ctttctgcat 60
   gttctccatt ccgctcctga tgtgcaggat tgcccagaat gcacgctaca ggaaaaccca 120
   ttcttctccc agccgggtgc cccaatactt cagtgcatgg gctgctgctt ctctagagca 180
   tatcccactc cactaaggtc caagaagacg atgttggtcc aaaagaacgt cacctcagag 240
   tccacttgct gtgtagctaa atcatataac agggtcacag taatgggggg tttcaaagtg 300
   gagaaccaca cggcgtgcca ctgcagtact tgttattatc acaaatctta a 351
<210> 2
   <211> 390
   <212> DNA
   <213> Homo sapiens
<400> 2 tactgctcct ttggtgaaat gaaagaataa 390
<210> 3
   <211> 27
   <212> DNA
   <213> Artificial Sequence
<220>
   <223> sense primer for human FSH alpha subunit
<400> 3
   agcgccatgg attactacag aaaatat 27
<210> 4
   <211> 37
   <212> DNA
   <213> Artificial Sequence
<220>
   <223> antisense primer for human FSH alpha subunit
<400> 4
   gggggatccg ggcccttaag atttgtgata attaaca 37
<210> 5
   <211> 33
   <212> DNA
   <213> Artificial Sequence
<220>
   <223> sense primer for human FSH beta subunit
<400> 5
   gggggcggcc gcaggatgaa gacactccag ttt 33
<210> 6
   <211> 27
   <212> DNA
   <213> Artificial Sequence
<220>
   <223> antisense primer for human FSH beta subunit
<400> 6
   ggggatatct tattctttca tttcacc 27
<210> 7
   <211> 592
   <212> DNA
   <213> Encephalomyocardit is virus
<400> 7
   gatatcgaat tccccctctc cctccccccc ccctaacgtt actggccgaa gccgcttgga 60
   ataaggccgg tgtgcgtttg tctatatgtt attttccacc atattgccgt cttttggcaa 120
   tgtgagggcc cggaaacctg gccctgtctt cttgacgagc attcctaggg gtctttcccc 180
   tctcgccaaa ggaatgcaag gtctgttgaa tgtcgtgaag gaagcagttc ctctggaagc 240
   ttcttgaaga caaacaacgt ctgtagcgac cctttgcagg cagcggaacc ccccacctgg 300
   cgacaggtgc ctctgcggcc aaaagccacg tgtataagat acacctgcaa aggcggcaca 360
   accccagtgc cacgttgtga gttggatagt tgtggaaaga gtcaaatggc tctcctcaag 420
   cgtattcaac aaggggctga aggatgccca gaaggtaccc cattgtatgg gatctgatct 480
   ggggcctcgg tgcacatgct ttacatgtgt ttagtcgagg ttaaaaaaac gtctaggccc 540
   cccgaaccac ggggacgtgg ttttcctttg aaaaacacga tgataaatgg cc 592
<210> 8
   <211> 654
   <212> DNA
   <213> Cytomegalovirus
<220>
   <221> promoter
   <222> (1)..(654)
<400> 8
   gatgtacggg ccagatatac gcgttgacat tgattattga ctagttatta atagtaatca 60
   attacggggt cattagttca tagcccatat atggagttcc gcgttacata acttacggta 120
   aatggcccgc ctggctgacc gcccaacgac ccccgcccat tgacgtcaat aatgacgtat 180
   gttcccatag taacgccaat agggactttc cattgacgtc aatgggtgga ctatttacgg 240
   taaactgccc acttggcagt acatcaagtg tatcatatgc caagtacgcc ccctattgac 300
   gtcaatgacg gtaaatggcc cgcctggcat tatgcccagt acatgacctt atgggacttt 360
   cctacttggc agtacatcta cgtattagtc atcgctatta ccatggtgat gcggttttgg 420
   cagtacatca atgggcgtgg atagcggttt gactcacggg gatttccaag tctccacccc 480
   attgacgtca atgggagttt gttttggcac caaaatcaac gggactttcc aaaatgtcgt 540
   aacaactccg ccccattgac gcaaatgggc ggtaggcgtg tacggtggga ggtctatata 600
   agcagagctc tctggctaac tagagaaccc actgcttaac tgcttatcga aatt 654
<210> 9
   <211> 441
   <212> DNA
   <213> Artificial Sequence
<220>
   <223> Adenovirus tripartite leader sequence
<400> 9
   tcgatactct cttccgcatc gctgtctgcg agggccagct gttgggctcg cggttgagga 60
   caaactcttc gcggtctttc cagtactctt ggatcggaaa cccgtcggcc tccgaacggt 120
   actccgccac cgagggacct gagcgagtcc gcatcgaccg gatcggaaaa cctctcgact 180
   gttggggtga gtactccctc tcaaaagcgg gcatgacttc tgcgctaaga ttgtcagttt 240
   ccaaaaacga ggaggatttg atattcacct ggcccgcggt gatgcctttg agggtggccg 300
   cgtccatctg gtcagaaaag acaatctttt tgttgtcaag cttgaggtgt ggcaggcttg 360
   agatctggcc atacacttga gtgacaatga catccacttt gcctttctct ccacaggtgt 420
   ccactcccag gtccaactgc a 441
<210> 10
   <211> 19
   <212> DNA
   <213> Artificial Sequence
<220>
   <223> sense primer for tripartite leader sequence
<400> 10
   gatatcgata ctctcttcc 19
<210> 11
   <211> 27
   <212> DNA
   <213> Artificial Sequence
<220>
   <223> antisense primer for tripartite leader sequence
<400> 11
   gcgtcgacct gcagttggac ctgggag 27
<210> 12
   <211> 564
   <212> DNA
   <213> Artificial Sequence
<220>
   <223> dihydrofolate reductase
<400> 12
   atggttcgac cattgaactg catcgtcgcc gtgtcccaaa atatggggat tggcaagaac 60
   ggagacctac cctggcctcc gctcaggaac gagttcaagt acttccaaag aatgaccaca 120
   acctcttcag tggaaggtaa acagaatctg gtgattatgg gtaggaaaac ctggttctcc 180
   attcctgaga agaatcgacc tttaaaggac agaattaata tagttctcag tagagaactc 240
   aaagaaccac cacgaggagc tcattttctt gccaaaagtt tggatgatgc cttaagactt 300
   attgaacaac cggaattggc aagtaaagta gacatggttt ggatagtcgg aggcagttct 360
   gtttaccagg aagccatgaa tcaaccaggc cacctcagac tctttgtgac aaggatcatg 420
   caggaatttg aaagtgacac gtttttccca gaaattgatt tggggaaata taaacttctc 480
   ccagaatacc caggcgtcct ctctgaggtc caggaggaaa aaggcatcaa gtataagttt 540
   gaagtctacg agaagaaaga ctaa 564
<210> 13
   <211> 130
   <212> DNA
   <213> Simian virus 40
<220>
   <221> polyA_signal
   <222> (1)..(130)
<400> 13
   aacttgttta ttgcagctta taatggttac aaataaagca atagcatcac aaatttcaca 60
   aataaagcat ttttttcact gcattctagt tgtggtttgt ccaaactcat caatgtatct 120
   tatcatgtct 130
<210> 14
   <211> 232
   <212> DNA
   <213> Artificial Sequence
<220>
   <223> bovine growth hormone
<220>
   <221> polyA_signal
   <222> (1)..(232)
<400> 14
   ctagagctcg ctgatcagcc tcgactgtgc cttctagttg ccagccatct gttgtttgcc 60
   cctcccccgt gccttccttg accctggaag gtgccactcc cactgtcctt tcctaataaa 120
   atgaggaaat tgcatcgcat tgtctgagta ggtgtcattc tattctgggg ggtggggtgg 180
   ggcaggacag caagggggag gattgggaag acaatagcag gcatgctggg ga 232
<210> 15
   <211> 10
   <212> DNA
   <213> Artificial Sequence
<220>
   <223> BamH I linker
<400> 15
   cgggatcccg 10